Europäisches Patentamt

European Patent Office

Office européen des brevets

(11) Publication number: **0 418 208 A1**

# EUROPEAN PATENT APPLICATION

(21) Application number: 90850308.9

(51) Int. Cl.5: **A61M 1/10**

(22) Date of filing: 14.09.90

(30) Priority: 15.09.89 SE 8903040

(43) Date of publication of application:
20.03.91 Bulletin 91/12

(84) Designated Contracting States:
AT BE CH DE DK ES FR GB GR IT LI LU NL SE

(71) Applicant: **PHARMACIA AB**

**S-751 82 Uppsala(SE)**

(72) Inventor: **Löfsjögard Nilsson, Erling**
**Vitmaravägen 77**
**S-194 60 Upplands Väsby(SE)**
Inventor: **Inacio, Jorge**
**Björnstigen 105**
**S-171 72 Solna(SE)**

(54) Fluid pump with associated drive means.

(57) A fluid pump, particularly an extracorporeal blood pump, comprises a pump chamber (1) having an inlet opening (2) and an outlet opening (3) connected to a respective non-return valve (4, 5). The inlet (2) and outlet (3) are movable in relation to each other and connected to a drive means (10, 11, 12, 13) for periodic movement of the inlet and outlet alternately towards and away from each other, the interior volume of the pump chamber (1) being variable in accordance with the relative distance between the inlet and the outlet. The drive means (10, 11, 12, 13) are constructed in such a way that the separating force exerted between the outlet (3) and inlet (2) of the pump chamber (1), when the outlet and inlet are moved away from each other during the filling stroke of the pump chamber, can not exceed an amount corresponding to a predetermined maximum permissible pressure difference between the surrounding pressure and the interior of the pump chamber (1), and preferably also in in such a way that the contracting force exerted between the inlet (2) and the outlet (3) when they are moved towards each other during the discharge stroke of the pump chamber can not exceed a predetermined maximum value corresponding to a predetermined maximum permissible pressure on the outlet side of the pump chamber.

Fig. 1

## FLUID PUMP WITH ASSOCIATED DRIVE MEANS

The present invention relates to an improved fluid pump, particularly for connection to the blood circulatory system of a living being.

The pump according to the invention has thus primarily been developed for use as an extracorporeal blood pump in conjunction, for instance, with surgical operations, dialysis, oxygenation of the blood in patients having an impaired lung function, for circulatory support after a cardiac insufficiency or for victims of accidents with heart or lung injuries, etc. It is also conceivable, however, for a pump according to the invention to be constructed in a manner which would enable it to be implanted in a patient as an artificial heart. It is also possible that a pump according to the invention could be advantageous to use also in other contexts, such as regional organ perfusion and/or organ perfusion in vitro.

Extracorporeal blood pumps used today for the above mentioned purposes are in the great majority of cases peristaltic so-called roller pumps, in which the blood is transported through a tube by rollers compressing the tube and simultaneously moving therealong. Recently a type of pulsatile pump has also begun to be used to a certain extent for the mentioned purposes, which pump basically consists of a pump bladder having flexible, elastic walls, which bladder has inlets and outlets provided with non-return valves and is enclosed in a surrounding rigid chamber, inside which a periodically varying pressure is generated for alternate compression and expansion of the pump bladder.

The peristaltic roller pumps have the serious drawback that it is very difficult to avoid considerable damages to the blood corpuscles in the pumped blood in consequence of the squeeze and shear forces that the blood corpuscles are subjected to in the pump.

The peristaltic roller pumps as well as the pulsatile pumps of the above mentioned type also have the common drawback that the operation of the pump will cause considerable subpressures at the inlet of the pump, and also in the pump bladder in the pulsatile pump, if the inflow of blood to the pump inlet does not correspond to the nominal pumped flow of the pump but falls below it. Such a situation is liable to occur with relative ease, for example as a result of a blockage at the end of the catheter connecting the pump to a blood vessel, for instance by abutment of the catheter end with the wall of the blood vessel. The subpressure which in the hitherto used pumps in such cases is automatically generated on the inlet side.of the pump and in the pump bladder may give rise to very troublesome problems. Thus, the subpressure may cause serious mechanical damages to the patient's blood vessels. Further, the subpressure may cause air to leak into the pump system through untight connections between various parts of the pump system, and if the subpressure is sufficient it may also result in gas being released from the pumped blood. In both cases serious gas emboli may occur. In the prior art blood pumps of the types discussed it is therefore necessary to monitor the pressure prevailing on the inlet side of the pump and in case of a subpressure intervene into the operation of the pump such that no serious situation may arise. Such a monitoring and control system will, of course, complicate and raise the price of the pump and will in itself constitute an additional source of possible malfunctions.

Both the peristaltic roller pumps and the pulsatile pumps of the aforesaid type further have the additional common drawback that the operation of the pump will create a considerable overpressure at the outlet from the pump, and also in the pump bladder itself in the case of the pulsatile pump, if the blood flow pumped out from the pump meets an abnormally increased resistance or a complete blockage. The overpressure which in such a case in the hitherto used pumps is automatically generated at the outlet from the pump and in the pump itself may reach very high values and give rise to serious problems. Thus, the overpressure may cause serious injuries to the patient connected to the pump. The overpressure may further be so high that the outlet tube from the pump to the patient and/or components forming part of the pump itself and/or connections between the different parts of the pump system will break. In the known blood pumps it is therefore necessary to monitor the pressure existing on the outlet side of the pump and in the event of an unpermissible overpressure intervene into the operation of the pump so that no serious situation may arise. Also this monitoring and control system will, of course, complicate and raise the price of the pump and will in itself constitute an additional source of possible malfunctions.

The basic construction and principle of a fluid pump of the type to which the present invention relates is described in WO 87/03492 (the disclosure of which is incorporated by reference herein).

The present invention thus relates to a fluid pump, particularly for connection to the blood circulatory system of a living being, of the type comprising a pump chamber having an inlet opening at one end and an outlet opening at the opposite end. A first non-return valve is connected to the inlet opening and permits a fluid flow only in the direction into the pump chamber through the inlet opening, and a second non-return valve is connected to the outlet opening and permits a fluid flow only in the direction out from the pump chamber through the outlet opening. The inlet and the outlet are movable in relation to each other in the direction of the pump chamber extension between the inlet and the outlet and are connected to controllable drive means for periodic displacement thereof alternately towards and away from each other in the direction of extension, the interior volume existing in the pump chamber between the inlet and outlet being variable in correspondence with the relative distance between them in the direction of extension.

In the case of a blood pump, such a pump may readily be constructed such that no mechanical damages will occur to the blood corpuscles in the pumped blood. The present invention is further based upon the discovery that in such a pump it is possible to automatically ensure, without any particular monitoring and control system, that no subpressure can be generated at the pump inlet or in the pump itself and neither any unpermissible overpressure at the pump outlet or in the pump itself.

In accordance with a primary aspect of the present invention unpermissible subpressures are prevented by constructing the drive means arrangement for the fluid pump in such a mechanical way that the separating force exerted between the outlet and the inlet of the pump chamber, when the outlet and the inlet are moved in the direction away from each other during the filling stroke of the pump chamber, can not exceed an amount corresponding to a predetermined maximum permissible pressure difference between the surrounding pressure (which e.g. may be the atmospheric pressure or the ambient pressure in a pressure chamber if the patient is subjected to such treatment) and the interior of the pump chamber. Thereby the generation of any excessive uncontrolled negative pressure within the pump chamber will be prevented.

In accordance with another aspect of the present invention unpermissible overpressures are prevented by constructing the the drive means for the blood pump in such a mechanical way that the contracting force exerted between the inlet and the outlet of the pump chamber, when the outlet and the inlet are moved in the direction towards each other during the discharge stroke of the pump chamber, can not exceed a predetermined maximum value corresponding to a predetermined maximum permissible pressure on the outlet side of the pump chamber.

The invention will now be described in more detail with reference to the accompanying drawings which by way of example illustrate some preferred embodiments of a blood pump according to the invention, and wherein

Fig. 1 schematically illustrates an axial section through one embodiment of a blood pump according to the invention at the end of a filling stroke for the pump chamber;

Fig. 2 schematically illustrates an axial section corresponding to that in Fig. 1 but at the end of a discharge stroke for the pump chamber;

Fig 3 illustrates an axial section through another embodiment of a blood pump according to the invention at the end of a filling stroke for the pump chamber;

Fig. 4 illustrates an axial section corresponding to that in Fig. 3 but at the end of a discharge stroke for the pump chamber;

Fig 5 illustrates an axial section through still another embodiment of a blood pump according to the invention at the end of a filling stroke for the pump chamber; and

Fig. 6 illustrates an axial section corresponding to that in Fig. 5 but at the end of a discharge stroke for the pump chamber.

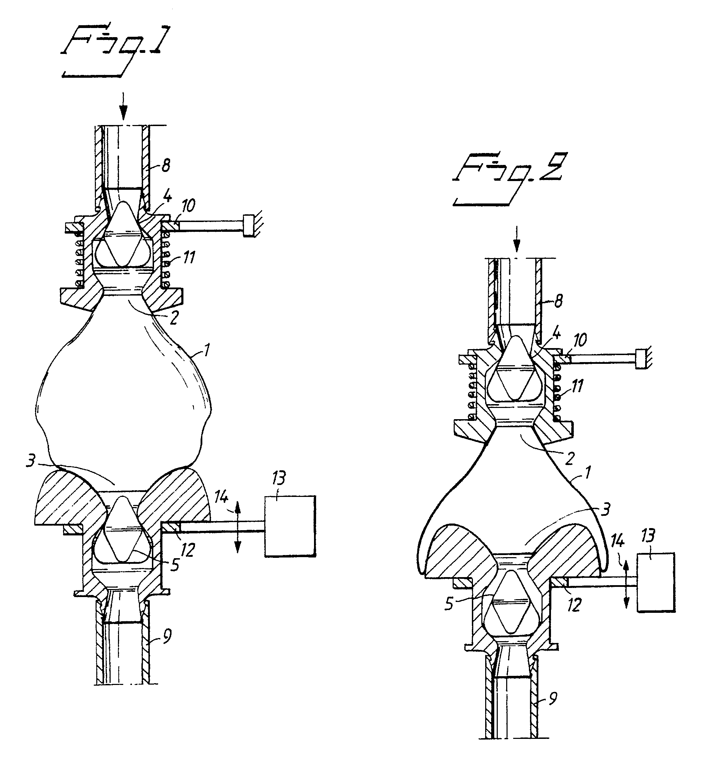

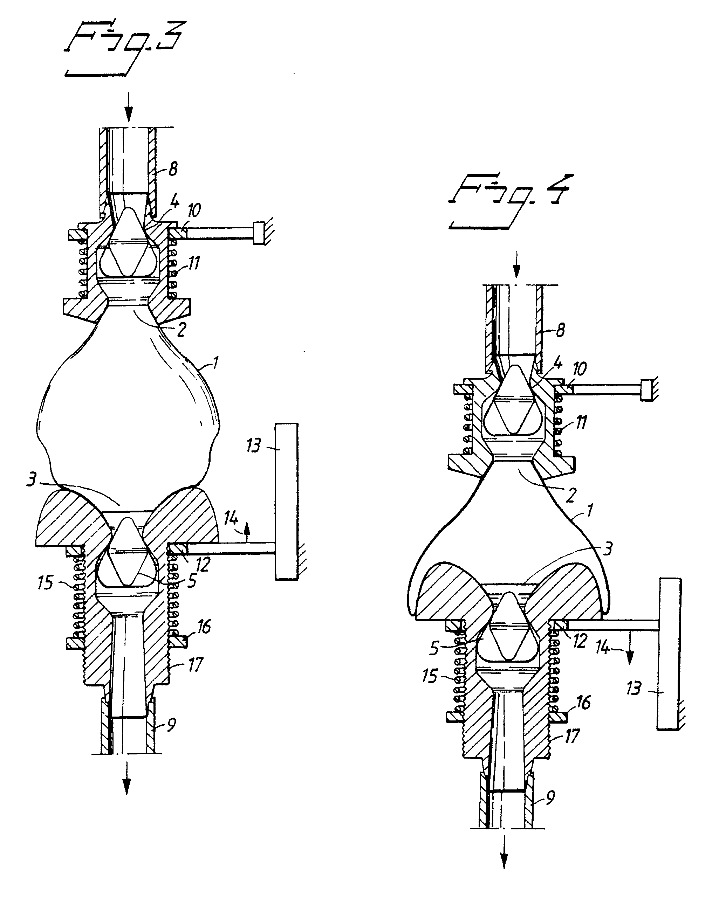

The pump according to the invention illustrated schematically and by way of example in Figs. 1 and 2 comprises a pump chamber 1 having an inlet 2 and an outlet 3 situated opposite to the inlet 2. Non-return valves 4 and 5 are connected to the inlet 2 and outlet 3, respectively, e.g. by gluing or ultrasonic welding, or by the valves being integral with the pump chamber. The valves 4 and 5 may, of course, be connected to the respective inlet by any other suitable means, such as, for example, a mechanical compression type connection or the like. The two valves are arranged such that the inlet valve 4 only permits a flow into the pump chamber 1, while the outlet valve 5 only permits a flow out from the pump chamber 1. In the illustrated embodiment the two non-return valves consist, as schematically shown, of movable valve bodies, which are acted upon by a closing force in a direction to sealing abutment against an associated valve seat, which closing force thus must be overcome for the valve to open. The closing force acting upon the valve body may, for example, consist of a spring force, or of the weight of the valve body if the valve body has a higher density than the pumped blood fluid, or as in the illustrated embodiment of the boyancy of the valve body in the pumped blood fluid as a result of the valve body having a lower density than the blood fluid, or, finally, simply result from the blood pressure on the valves in the flow direction during the systolic (compression) and diastolic (relaxing) periods, respectively.

The inlet 2 with the inlet valve 4 and the outlet 3 with the outlet valve 5 are reciprocatingly movable in relation to each other between the most brought apart position illustrated in Fig. 1 and the most brought together position illustrated in Fig. 2 by a drive means described in more detail hereinafter. In the illustrated embodiment the pump chamber 1 is constructed as a substantially spherical bladder having flexible but substantially non-stretchable walls and which is constructed in such manner as to enable a part of its wall to be folded telescopically from the state shown in Fig. 1 to the state shown in Fig. 2 when the inlet 2 with the inlet valve 4 and the outlet 3 with the outlet valve 5 are moved in a direction towards each other from the greatest distance between them shown in Fig. 1 to the smallest distance shown in Fig. 2. This may, for example, be achieved by the lower part of the pump chamber 1 having a reduced wall thickness as compared to the remaining part thereof or by the upper part of the pump chamber being provided with a supporting collar. The pump chamber or the interior volume of the pump bladder then varies in accordance with the varying distance between the inlet 2 and the outlet 3.

The pumping normally takes place in such a manner that when the inlet 2 and the outlet 3 by means of the drive means described below are moved away from each other from the position shown in Fig. 2 to the position shown in Fig. 1, the volume of the pump chamber 1 is increased and blood flows into the pump chamber 1 through the now open inlet valve 4 from the inlet tube 8 connected to the patient's blood circulatory system. This is accordingly the filling stroke of the pump chamber 1. When the inlet 2 and the outlet 3 by means of the drive means described below are then moved in a direction towards each other from the position shown in Fig. 1 to the position shown in Fig. 2, the volume of the pump chamber 1 is decreased so that blood is pressed out from the pump chamber through the now open outlet valve 5 to the outlet tube 9 connected to the patient's blood circulatory system. This is accordingly the discharge stroke of the pump chamber 1. It will be appreciated that the blood volume pumped per unit of time is determined by the frequency and the magnitude of the relative reciprocating movement between the inlet 2 and the outlet 3.

In the case of the exemplifying embodiment, illustrated in the drawings, of the drive means for the pump according to the invention, the inlet 2 with the inlet valve 4 is arranged in an annular holder 10 which is stationarily mounted in a suitable manner not shown in any detail. The inlet 2 with the inlet valve 4 is axially movable in relation to the holder 10 through a limited distance corresponding to the length of the maximum desired relative movement between the inlet 2 and the outlet 3. Further, a spring means 11, only illustrated schematically and the spring force of which preferably is adjustable, is arranged between the holder 11 and the inlet 2 with the inlet valve 4. The outlet 3 with the outlet valve 5 is similarly arranged in an annular holder 12 and is axially movable in relation to this holder 12 through a distance corresponding to the length of the maximum desired relative movement between the inlet 2 and the outlet 3. The holder 12 is movable in the axial direction of the pump chamber 1 and connected to a suitable drive mechanism 13, only illustrated schematically and not in any detail, by which drive mechanism the holder 12 may be forcibly driven reciprocatingly by a suitable drive motor as is indicated by an arrow 14 with a desired frequency and with a distance of movement corresponding to the maximum desired relative displacement between the inlet 2 and the outlet 3.

The described drive means for the pump operates in the following way. When the holder 12 during a filling stroke for the pump chamber 1 is forcibly moved by the drive mechanism 13 in the direction downwards from the position shown in Fig. 2 to the position shown in Fig. 1, the outlet 3 with the outlet valve 5 will normally accompany the downward movement of the holder 12 under the influence of its own weight and the weight of the blood present in the pump chamber 1. This total weight, which optionally may be assisted by an additional defined and predetermined spring force as will be described in more detail below, is adapted not to substantially exceed the necessary opening pressure for the inlet valve 4. The latter is accordingly opened and blood flows into the pump chamber 1 from the inlet tube 8 so that the volume of the pump chamber 1 is increased and the outlet 3 with the outlet valve 5 can accompany the holder 12 in the downward movement thereof. It will be realized in this context that the filling pressure for the pump chamber, which must be greater than the necessary opening pressure of the inlet valve, may be adjusted by varying the level of the pump chamber in relation to the patient. It will, however, be appreciated that blood may flow into and fill the pump chamber 1 in this manner only to the extent as blood is supplied through the inlet tube 8. If the blood supply through the inlet tube is too small, the outlet 3 with the outlet valve 5 will not be able to accompany the downward movement of the holder 12 to the full extent. If the blood supply through the inlet tube 8 is completely stopped, the outlet 3 with the outlet valve 5 will remain in the position illustrated in Fig. 2, while the holder 12 is moved downwards by the drive mechanism 13. It will be appreciated that the subpressure that may arise in the inlet tube 8 in relation to the ambient (usually atmospheric) pressure can never exceed a value corresponding to the weight of the outlet 3 with the outlet valve 5 and of the blood quantity that is present in the pump chamber 1 in the position illustrated in Fig. 2. The pump will consequently never strive to pump a larger quantity of blood than is supplied through the inlet tube 8, and if this blood supply is interrupted completely, the effective pumping also stops automatically without the necessity of influencing the driving of the holder 12 by means of the drive mechanism 13 in any way and without permitting any uncontrolled substantial subpressure to be generated in the inlet tube 8 or in the pump chamber 1. If the blood supply through the inlet tube 8 is interrupted completely, the outlet 3 with the outlet valve 5 and the pump chamber 1 will remain in the state illustrated in Fig. 2, while the holder 12 may be reciprocated by means of the drive mechanism 13.

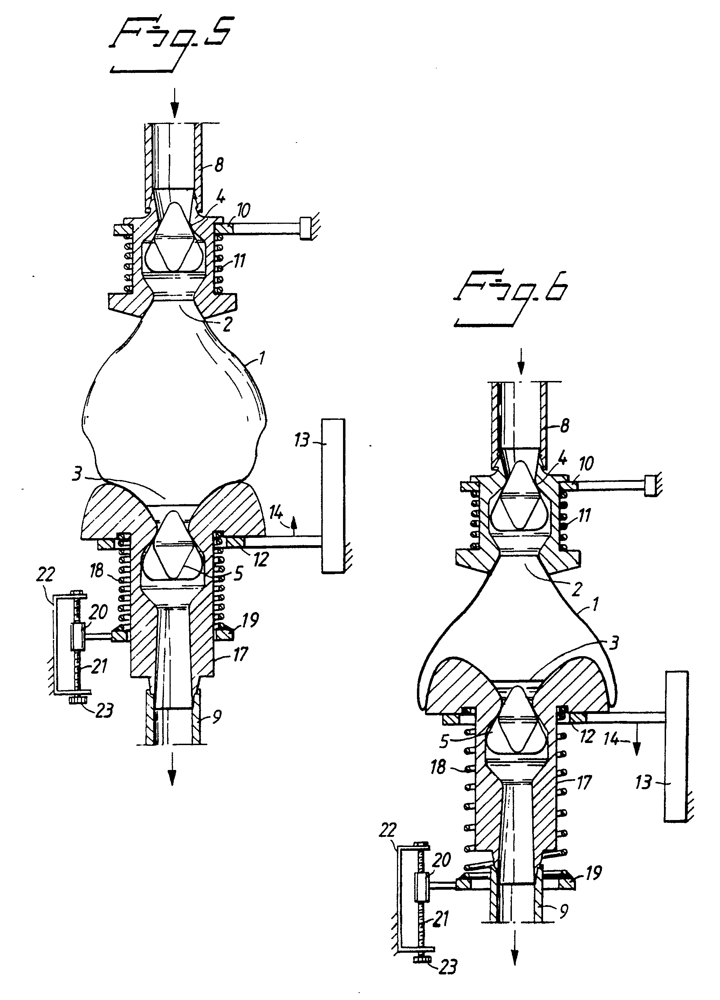

The relative separating force between the inlet 2 and the outlet 3, which in the illustrated embodiment is generated by the weight of the outlet 3 with the outlet valve 5 and the blood in the pump chamber 1, may naturally also and equally well be generated by means of a spring member acting between the inlet 2 and the outlet 3. This may in particular be suitable if the pump is oriented in another way than in the embodiment illustrated, such as horizontally. It is, of course, also possible to balance the aforesaid gravity completely or partially with an opposing spring force, if suitable. Examples of such embodiments of the pump are illustrated in Figs. 3 to 6 and will be described further on.

When during a discharge stroke for the pump chamber 1 the holder 12 is driven upwards by the drive mechanism 13 from the position illustrated in Fig. 1 to the position illustrated in Fig. 2, the inlet 2 with the inlet valve 4 is normally stationary due to a suitably adjusted bias of the spring 11, and the volume of the pump chamber 1 is therefore reduced from that shown in Fig. 1 to that shown in Fig. 2 and blood is pressed out from the pump chamber 1 through the outlet valve 5 to the outlet tube 9. Should the resistance to the blood flow through the outlet tube 9 then be so great that the pressure in the pump chamber 1 will tend to exceed the biasing force of the spring means 11, this spring means will begin being compressed and the inlet 2 with the inlet valve 4 will therefore begin to move upwards relative to the stationary holder 10. The pressure in the pump chamber 1, and thereby in the outlet tube 9 in the course of the discharge stroke for the pump chamber, may thus never exceed a value corresponding to the spring force of the spring means 11. If there is a complete blockage of the blood flow in the outlet tube 9, the inlet 2 with the inlet valve 4 will completely accompany the movement of the outlet 3 with the outlet valve 5 while being driven by the drive mechanism 13, whereby the effective pumping ceases completely without the necessity of influencing the driving of the holder 12 by means of the drive mechanism 13 and without the overpressure in the pump chamber 1 and thereby the outlet tube 9 exceeding a value corresponding to the spring force of the spring means 11 in the most compressed state thereof. The spring means 11 is thus suitably constructed in such a manner that its bias in the non-compressed state corresponds to the highest pressure that should normally exist in the outlet tube 9 and that its maximum spring force in the maximally compressed state corresponds to the highest overpressure in the outlet tube 9 that can be permitted. It will be understood that the spring means 11 may be constructed in such a manner as to enable both its biasing force and its maximum spring force as well as its characteristic between these two values to be varied or set depending on the use in question of the pump, for example depending on which circulatory system of a patient that the pump is to be connected to. It is to be noted that the illustrated location of the spring means 11 is only exemplary and for ease of illustration and that the same function will be obtained if the spring means are arranged otherwise, e.g in the actual drive mechanism.

Reference is now made to Figs. 3 to 6 wherein corresponding parts are provided with the same reference designations as before. Figs. 3 and 4 illustrate the pump according to Figs. 1 and 2 modified by the addition of a spring means 15 acting between the holder 12 and a support 16 attached to an extended outlet portion 17 of the pump chamber. The support 16 is preferably a nut member threadedly engaged with the outlet portion 17 to permit the spring means to be suitably biased. When the holder 12 is moved downwards from the position illustrated in Fig. 4 to the position illustrated in Fig. 3, the outlet 3 with the outlet valve 5 will normally accompany the downward movement of the holder 12. Should, however, e.g. due to a decrease or blockage of the inflow of blood to the inlet 2, a subpressure tend to be created in the pump chamber 1, the spring means 15 will be compressed and the outlet 3 remain stationary. The maximum subpressure that may be created in the pump chamber is thus determined by the spring force of the spring means when the latter is in its most compressed state, i.e. when the holder 12 is in its lowest position. This spring force may be suitably set by adjusting the vertical position of the nut member 16.

Figs. 5 and 6 illustrate a variation of the embodiment in Figs. 3 and 4 wherein the separating force acting upon the outlet portion of the pump chamber is totally independent of the movement of the holder 12. In this embodiment a spring means 18 is attached at its upper end to the outlet part 3

of the pump chamber 1 and at its lower end to an annular support 19 surrounding the extended outlet portion 17 and freely movable in relation thereto. The support 19 is in turn fixed to a nut member 20 threadedly engaged with a screw 21 rotatably mounted in a stationary bracket 22. Through vertical adjustment of the nut member 20 by rotation of the screw head 23 of the screw 21, the spring means 18 may be biased as desired. When the holder 12 is moved downwards from the position shown in Fig. 6 to the position shown in Fig. 5, the spring means 15, as adjusted in the figures, will successively contract exerting a defined separating force between the inlet 2 and the outlet 3. The maximum subpressure created thereby in the pump chamber will correspond to the maximum spring force, and a desired maximum subpressure level in the pump chamber may thus be set by adjustment of the vertical position of the support 19. It will be understood that the spring means 18 may also, if desired, be adjusted to partially or completely balance the gravitational force of the outlet 3 with the outlet valve 5 and the blood quantity present in the pump chamber 1.

With reference to all the embodiments described above and presented in the drawings it will be understood that the described function of the pump will also be obtained if the holder 12 for the outlet 3 with the outlet valve 5 is stationary, while the holder 10 for the inlet 2 with the inlet valve 4 is instead forcibly reciprocated by means of the drive mechanism 13. The same functional mode will also be obtained if the two holders 10 and 12 are movable and reciprocated by means of a common drive mechanism or individual drive mechanisms, the sum of the distances of movement of the two holders corresponding to the maximally desired relative displacement between the inlet 2 and the outlet 3 of the pump chamber 1. The movement distance of each holder may, for example, correspond to half the maximally desired displacement between the inlet and the outlet. In the case of such a construction of the drive means the magnitude of the pumped flow may be varied, not only by varying the frequency and stroke length of the driving, but also by changing the relative phase position of the drivings of the two holders 10, 12, a maximum pumped flow being obtained when the holders 10, 12 move in counter-phase, while the effective pumping will be zero if the holders 10 and 12 are driven in phase with each other.

It will be understood from the aforegoing that if the effective pumping substantially ceases in the manner described above due to the blood supply through the inlet tube 8 being choked or the blood flow out through the outlet tube 9 being choked, the blood present in the pump chamber 1 and the non-return valves will in spite thereof be maintained in a certain movement as a result of the continued operation of the pump by means of the drive mechanism 13. This is of essential importance to prevent coagulation of the blood. It will also be appreciated that the pump according to the invention also involves the very important advantage that when the pump is in use, for example in conjunction with a surgical procedure, the effective pumping can be stopped at any time by closing either the inlet tube 8 or the outlet tube 9 with a forceps, and then starting the pumping again by removing the forceps, without the operation of the pump by means of its drive means having to be influenced in any way and without any risk that neither unpermissible subpressures nor unpermissible overpressures may be created.

It will further be understood that the non-return valves do not necessarily have to be placed in immediate connection to the inlet and outlet, respectively, of the pump chamber, but could also be arranged at a distance from the pump chamber, for example in the inlet tube 8 and the outlet tube 9, respectively.

It is further to be mentioned that the present invention in a blood pump of the type in question, primarily relating to the construction of the drive means arrangement of the pump, without any disadvantage and rather with advantage may be combined with the invention disclosed in our patent application entitled "Blood pump with flexible pump chamber" and filed simultaneously herewith (the disclosure of which is incorporated by reference herein). The invention according to the present application and the invention according to said application filed simultaneously herewith, which relates to a pump having a collapsible pump chamber, will in an advantageous manner cooperate to further ensure that no unacceptable subpressure may be created on the inlet side of the pump or in the pump itself.

Therefore, in an advantageous embodiment of the present invention, the pump chamber is at least partially made of a flexible material having such a construction that under the influence of a pressure difference which is caused by the ambient pressure exceeding the interior pressure of the pump chamber and which is greater than a predetermined value, normally the opening pressure difference for the first non-return valve connected to the inlet, the pump chamber is collapsible in a controlled manner by a volume at least corresponding to the maximum increase of the interior volume of the pump chamber when the inlet and the outlet are moved away from each other.

The invention is, of course, not restricted to the embodiments specifically described above and illustrated in the drawings, and several changes and modifications may be made within the scope of the present inventive concept as defined in the following claims.

## Claims

1. A fluid pump, particularly for connection to the blood circulatory system of a living being, comprising a pump chamber (1) having an inlet opening (2) at one end thereof and an outlet opening (3) at the opposite end thereof, a first non-return valve (4) connected to the inlet opening and permitting a fluid flow only in the direction into the pump chamber through the inlet opening and a second non-return valve connected to the outlet opening (3) and permitting a fluid flow only in the direction out from the pump chamber through the outlet opening, the inlet and the outlet (2, 3) being movable in relation to each other in the direction of the extension of the pump chamber (1) between the inlet and the outlet (2, 3) and being connected to controllable drive means (10, 11, 12, 13) for periodic movement thereof alternately towards and away from each other in said direction of extension, the interior volume existing in the pump chamber (1) between the inlet (2) and the outlet (3) being variable in correspondence with the relative distance between them in said direction of extension, characterized in that said drive means arrangement has such a mechanical construction that the separating force exerted between the outlet (3) and the inlet (2) of the pump chamber (1), when the outlet (3) and the inlet (2) are moved in the direction away from each other during the filling stroke of the pump chamber (1), can not exceed an amount corresponding to a predetermined maximum permissible pressure difference between the the surrounding pressure and the interior pressure of the pump chamber (1), thereby preventing the generation of any excessive uncontrolled negative pressure within the pump chamber (1).

2. A pump according to claim 1, characterized in that said predetermined maximum permissible pressure difference is selected such that no substantial gas release from the pumped fluid will be generated.

3. A pump according to claim 1 or 2, characterized in that said drive means arrangement (10, 11, 12, 13) further has such a mechanical construction that the contracting force exerted between the inlet (2) and the outlet (3) of the pump chamber (1), when the outlet (3) and the inlet (2) are moved in the direction towards each other during the discharge stroke of the pump chamber (1), can not exceed a predetermined maximum value corresponding to a predetermined maximum permissible pressure on the outlet side of the pump chamber (1).

4. A pump according to claim 1, 2 or 3, characterized in that the inlet (2) and the outlet (3) of the pump chamber are acted upon by a relative separating force independent of the relative movement and operation of the drive means (10, 11, 12, 13).

5. A pump according to claim 4, characterized in that said separating force is a gravitational force.

6. A pump according to claim 4, characterized in that said separating force is or includes a spring force.

7. A pump according to claim 6, characterized in that said spring force is generated by a spring means (18) acting between the outlet part (3) of the pump chamber (1) and a stationary part (22) of the pump.

8. A pump according to claim 7, characterized in that said spring force is adjustable.

9. A pump according to claim 1, 2 or 3, characterized in that the separating force exerted between the inlet (2) and the outlet (3) of the pump chamber (1) during the filling stroke of the pump chamber is provided by said drive means (12) acting upon the outlet part (3, 16, 17) of the pump chamber via an intermediate compression spring means (15) such that the maximum separating force that may be exerted is restricted to the spring force of said spring means.

10. A pump according to any one of claims 1 to 9, characterized in that said drive means comprise two drive elements (10, 12) forcibly reciprocatable in relation to each other in the direction of extension of the pump chamber (1), the relative distance of displacement of said drive elements corresponding to the maximum desired relative displacement between the inlet (2) and outlet (3) of the pump chamber, and said two drive elements (10, 12) being connected to the inlet (2) and outlet (3), respectively, of the pump chamber in such a way that the inlet and the outlet, respectively, are restrictedly movable in relation to the respective drive element through a distance having a length corresponding to the maximum desired relative displacement between the inlet and outlet; and that spring means (11) are arranged in such manner that said spring means are compressed if the contracting force exerted between the inlet (2) and outlet (3) of the pump chamber during a discharge stroke of the pump chamber tends to exceed a predetermined value and thereby restricts said contracting force to correspond to the spring force of said spring means (11).

11. A pump according to claim 10, characterized in that said spring means (11) are coupled into the force transmitting path between one of said drive elements (10) and the part (2) of the pump chamber (1) which is connected thereto.

12. A pump according to claim 10 or 11, characterized in that said spring means (11) are biased.

13. A pump according to claim 10, 11 or 12, characterized in that the spring force of said spring means (11) is adjustable.

14. A pump according to any one of claims 10 to 13, characterized in that compression spring means (15) are mounted between the outlet (3) of the pump chamber (1) and the drive element (12) connected to said outlet (3), said spring means optionally being biased.

15. A pump according to claim 14, characterized in that the spring force of said spring means (15) is adjustable.

16. A pump according to any one of claims 10 to 15, characterized in that one drive element (10) is stationary while the other drive element (12) is forcibly reciprocatable through a distance corresponding to the maximum desired relative displacement between the inlet (2) and outlet (3) of the pump chamber.

17. A pump according to any one of claims 10 to 15, characterized in that both the drive elements (10, 12) are movable and forcibly reciprocatable, the sum of the movement distances of the two drive elements corresponding to the maximum desired relative displacement between the inlet (2) and outlet (3) of the pump chamber.

Fig.1

Fig.2

Fig.3

Fig.4

Fig.5

Fig.6

| | DOCUMENTS CONSIDERED TO BE RELEVANT | | EP 90850308.9 |
|---|---|---|---|
| Category | Citation of document with indication, where appropriate, of relevant passages | Relevant to claim | CLASSIFICATION OF THE APPLICATION (Int. Cl.5) |
| D,A | WO - A1 - 87/03 492 (DATA PROMEDITECH I.N.C. AKTIEBOLAG) * Page 7, paragraph 3 - page 9, paragraph 2; fig. 2,3 * | 1 | A 61 M 1/10 |
| A | US - A - 4 058 855 (RUNGE) * Totality * | | |

TECHNICAL FIELDS
SEARCHED (Int. Cl.5)

A 61 M 1/00

The present search report has been drawn up for all claims

| Place of search | Date of completion of the search | Examiner |
|---|---|---|
| VIENNA | 21-12-1990 | VELINSKY-HUBER |